# EUROPEAN PATENT SPECIFICATION

(11) **EP 1 727 886 B1**
(45) Date of publication and mention of the grant of the patent: **30.01.2008**
(21) Application number: 05717047.4
(22) Date of filing: 15.03.2005
(51) Int. Cl.: C11D 3/39, C07D 209/48

(54) **PROCESS FOR OBTAINING GRANULAR COMPOSITIONS**
VERFAHREN ZUR HERSTELLUNG VON GRANULATFÖRMIGEN ZUSAMMENSETZUNGEN
PROCÉDÉ POUR L'OBTENTION DE COMPOSITIONS GRANULAIRES

(30) Priority: 16.03.2004 IT MI20040498
(43) Date of publication of application: 06.12.2006
(73) Proprietor: Solvay Solexis S.p.A., 20121 Milano (IT)
(72) Inventor: BIANCHI, Ugo Piero, I-37126 VERONA (IT); GARAFFA, Roberto, I-20141 MILANO (IT)
(74) Representative: Vande Gucht, Anne
(86) International application number: PCT/EP2005/051173
(87) International publication number: WO 2005/090543

(56) References cited:
- EP-A- 0 816 481
- EP-A- 0 852 259
- US-A- 5 279 757
- US-A- 5 419 846
- US-A- 5 480 577

## Description

The present invention relates to a process for obtaining granular compositions based on solid peroxycarboxylic acids (peracids) with improved bleaching efficacy, which may be used even at moderate temperature also, of the order of from 10°C to 30°C for industrial and commercial applications in detergency and disinfection.

More particularly, the invention relates a process for obtaining granules based on sparingly water-soluble peracids, for instance imidoalkanepercarboxylic acids, preferably ε-phthalimidoperoxyhexanoic acid, referred to hereinbelow as PAP, the said peracids being supported, as described below, and the said granules having in the washing bath at a temperature of 20°C a dissolution time t₉₀, as defined below, of less than ten minutes and preferably less than five minutes.

These granules may be used, in particular for bleaching, in detergent compositions, especially in powder form or in the form of tablets.

The time t₉₀ is defined as the time required for 90% of the peracid contained in the granules introduced into the washing bath to dissolve in the bath at a temperature of 20°C. A standard washing bath, as described in the examples, is used to measure this parameter.

It is known that detergent compositions are complex and are obtained by combining numerous components. The said compositions are often obtained from components in granular form, to avoid problems of pollution, dusting and irritation during their production and use. In particular, in the case of highly active ingredients such as peracids, the said granular form allows these active principles and the other components present to remain unchanged, since the interaction of the peracids with the other components of the composition is limited. It is also known that the bleaching and disinfecting efficacy of the peracids depend not only on the chemical composition of the products and on the conditions adopted for washing operations, but also on the chemical composition of the granular compositions containing the said peracids. In the case of sparingly water-soluble peracids, the efficacy depends also on the characteristics of the peracid particles present in the said granules. The properties of the granules become even more critical when it is desired to use the detergent composition under particularly mild application conditions, for example in cold water, for example at a temperature of 20°C, and for short washes, of even less than 30 minutes, as is required by the new market demands.

US 5,480,577 discloses wax-encapsulated core particles used in liquid detergent compositions, the core of the particles comprising a bleaching agent, such as an organic peroxy acid (e.g. a PAP), and 0.01 to 5% of surfactant which aids in the dispersion of the waxy coating to promote more rapid dissolution of the bleaching compound.

Patent EP 852 259 in the name of the Applicant describes PAP-based granular compositions with optimum characteristics in terms of activity (high content of active principle); stability over time (shelf life) even under severe conditions; bleaching efficacy and disinfecting efficacy; safety, even in the case of accidental exposure to overheating; not to mention processability even on an industrial scale. Tests performed by the Applicant have shown that the granular compositions of the said patent are not of satisfactory efficacy when they are used in detergency and in disinfection under mild application conditions, for example at a temperature of 20°C, and for short washes, of less than or equal to 30 minutes. The granular compositions described in EP 852 259 have dimensions of about 0.25 mm to about 1.4 mm. Tests performed by the Applicant have shown that the dissolution times of these PAP-containing granules are of the order of 30 minutes to obtain a dissolution of 90% by weight of the peracid, at a temperature of 20°C. Dissolution times of this order of magnitude are unsatisfactory for the granular compositions that the market requires for short washes, of even less than 30 minutes, and at very low temperatures, for example at 20°C as indicated above.

It was thus found that there is a need for granular compositions based on active imidoalkanepercarboxylic acids that are effective under mild application conditions, for example in water at a temperature of from 10°C to 30°C, and for short washes, of even less than 30 minutes, the dissolution time t₉₀ in the washing bath, at a temperature of 20°C, of the imidoalkanepercarboxylic acids present in the granular compositions being less than ten minutes and preferably less than five minutes.

The Applicant has found, surprisingly and unexpectedly, a process for obtaining granular compositions containing imidoalkanepercarboxylic acids, which solve the technical problem indicated above.

One subject of the present invention is a process for obtaining granular compositions of imidoalkanepercarboxylic acids in β form, comprising the said peracids in an amount of from 5% to 80% by weight and preferably from 30% to 70% by weight; the said peracids being supported on supports with hydrophilic or hydrophobic characteristics; the said granular compositions being from 0.25 mm to 1.4 mm in size and having a dissolution time t₉₀ of the peracid at 20°C of less than ten minutes and preferably less than five minutes; the sum of the components indicated above and of those optionally present being 100%, comprising the following steps :
I) preparation of an aqueous dispersion containing an amount of imidoalkanepercarboxylic acids, expressed as a percentage by weight, of from 20% to 70%, working at temperatures of between 20°C and 65°C;
II) deposition of the imidoalkanepercarboxylic acids present in the aqueous dispersion prepared in step I) onto the support agent, preferably by impregnation or spraying, using a fluid-bed drier, working at temperatures of between 10°C and 65°C and at a pressure of between 10 mm Hg and 800 mm Hg, to obtain an aggregate;
III) optionally, granulation of the aggregates obtained in step II), optional addition of other additives, and drying of the granules obtained;
IV) optionally, screening of the dried granules, followed by coating of the screened granules and drying of the coated granules;
V) screening of the granules obtained in step II) if the optional steps III) and IV) are not performed, or in the optional step III) if the optional step IV) is not performed, or in the optional step IV), by selecting the granulometric fraction of between 0.25 mm and 1.4 mm.

The imidoalkanepercarboxylic acids, preferably have the formula (I) 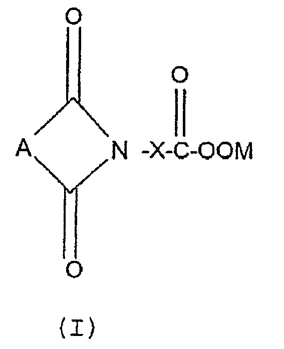
in which A indicates a group chosen from the following: 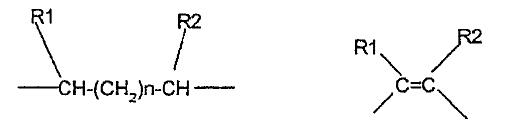 or 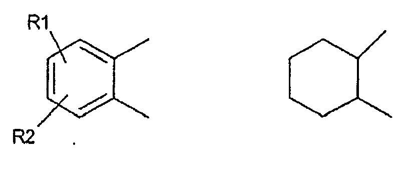 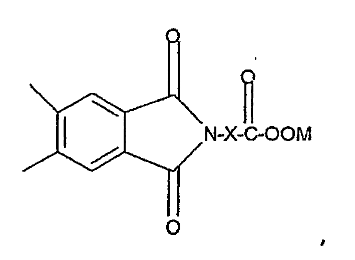 in which:
n is an integer 0, 1 or 2,
R1 has one of the following meanings: hydrogen, chlorine, bromine, C₁-C₂₀ alkyl, C₂-C₂₀ alkenyl, aryl or alkylaryl,
R2 is hydrogen, chlorine, bromine or a group chosen from the following: -SO₃M, -CO₂M, -CO₃M or -OSO₃M,
M means hydrogen, an alkali metal, ammonium or an equivalent of an alkaline-earth metal,
X indicates a C₁-C₁₉ alkylene or an arylene;
the said imidoalkanepercarboxylic acids being in the "β-crystal" form and having a dissolution time t₉₀ in the washing bath at a temperature of 20°C of less than ten minutes, preferably less than five minutes and even more preferably less than 3.5 minutes.

The imidoalkanepercarboxylic acid is preferably ε-phthalimidoperoxy-hexanoic acid (PAP).

The said dissolution time t₉₀ is determined by dispersing a sample of the granular composition (granulometric fraction of from 0.25 mm to 1.4 mm) in a solution prepared with water with a hardness of 10°F (French degrees) and containing a standard detergent base, being free of bleaching additives (IEC detergent type B, with phosphates - IEC publication 60456) the system being kept stirred and thermostatically maintained at a temperature of 20°C; the times at which the samples are taken, measured from the moment of mixing of the two compositions, are plotted on a graph on the x-axis, and the areas of the peracid peak, determined by HPLC chromatographic analysis, are plotted on the y-axis; the time t₉₀ at which the amount of dissolved peracid corresponds to 90% of the peracid present in the system, which is determined from the peracid concentration obtained asymptotically on this graph at infinite time (100%), is derived from a graph thus obtained. A detailed description of the method is given in the examples.

The crystalline form of the imidoalkanepercarboxylic acids referred to herein as the β form is the form known in the art for the said peracids, which may be obtained directly by crystallization of the said compounds.

The imidoalkanepercarboxylic acids may be obtained via methods known in the art: see, for example, European patents EP 325 288, EP 325 289, EP 490 409, EP 556 769, EP 560 155 and EP 780 374 in the name of the Applicant.

Imidoalkanepercarboxylic acids in β form obtained from the corresponding α form of these peracids, as described in the patent application WO 2004/007452 in the name of the Applicant, are preferably used in the present invention.

The supports used in the granules obtained by the process of the invention carry (carriers) the imidoalkanepercarboxylic acids. The said supports are preferably substances that are at least partially soluble in the washing bath.

The dimensions of the supports are from about 0.01 mm to 0.35 mm and preferably from about 0.05 mm to 0.25 mm.

Examples of supports are natural or synthetic polymers; the said supports preferably consist of cellulose, for example cellulose fibres or methylcellulose, optionally polysaccharides and optionally expanded starch, polyvinylpyrrolidone, and natural gums, for example guar gum. Other examples of supports are also metal oxides or sulfates, for example magnesium or aluminium oxide or sulfate, zeolites and silicas.

Solid components that are added to the composition to control the exothermicity (phlegmatizers), which are described below, may also optionally be used as supports.

Cellulose fibres, polysaccharides and amides are preferably used as supports. In general, the cellulose fibres are from 0.05 to 0.40 mm long and the diameter is generally about 10 micrometres.

Preferably, the weight ratio between the peracids and the support in the granular compositions of the invention is between 0.1 and 4.0, the sum of the components present in the granular composition being 100%.

The granular compositions obtained by the process of the invention may also contain additional components for controlling the exothermicity (phlegmatizers), which arises in the event of undesired overheating. Examples of these additives are boric acid, water and salts of metals from groups IA, IIA and IIIA and of organic or mineral acids that are chemically compatible with the imidoalkanepercarboxylic acids and that can crystallize in hydrated form. Mention may be made in this respect of magnesium sulfate pentahydrate, calcium lactate hydrate and calcium sulfate dihydrate; boric acid is preferred. In general, these products are used in an amount of between 3.5% and 35% by weight.

Other components that may be added and that have the function of blocking the catalytic action for decomposition of the imidoalkanepercarboxylic acids by the heavy metal ions are chelating agents and/or sequestering agents, in an amount of from 0.005% to 5% by weight relative to the total weight of the composition. Mention may be made of quinoline and salts thereof, alkali metal polyphosphates, picolinic acid and dipicolinic acid, and monophosphonic or polyphosphonic acids, for example preferably 1-hydroxyethylidene-1,1-diphosphonic acid (HEDP).

Other components are water-soluble binders of polymer type, for instance acrylic acid polymers or copolymers of acrylic acid with maleic acid and/or anhydride, or copolymers of acrylic acid derivatives such as esters and salts; acrylic acid homopolymers are preferably used. These components have the function of giving the granule better mechanical properties; in general, they are used in amounts ranging from 0.1 % to 5% by weight relative to the total weight.

Other additional components are surfactants, preferably anionic and/or nonionic surfactants, or mixtures thereof, in amounts of from 0.1 % to 10% by weight relative to the total weight, and viscosifiers, which are used to prepare the initial aqueous suspensions of the imidoalkanepercarboxylic acids, in weight percentage amounts of from 0.001 % to 5% relative to the total weight of the composition.

Optionally, the surface of the granules obtained may be coated with film-forming agents, which are applied via methods known in the art. Examples of film-forming agents are polyacrylic acid, vinyl polymers, for instance polyvinylpyrrolidone, cellulose polymers, for instance methylcellulose and ethylcellulose, natural gums, for instance zein gum and gum shellac, acacia gum, gelatin, fatty acids, for instance myristic acid, paraffins, synthetic waxes, caprolactone, oligomers and polymers thereof.

Surfactants, preferably anionic and nonionic surfactants, antifoams and suspending agents may also be used in step I), in amounts such that they are within the limits as defined above.

Imidoalkanepercarboxylic acids in β-crystal form obtained from the corresponding α-crystal form, according to the process described in patent application WO 2004/007452 in the name of the Applicant, are preferably used in step I). In this case, nonionic surfactants are preferably used in step I).

Additives for controlling any exothermicity (phlegmatizers), chelating agents and/or sequestering agents, surfactants and binders may also be used in step II) and in step III).

The additives mentioned previously may also be used in the optional step IV).

The amounts of the additives added in the various steps of the process must be such that they respect the limits indicated above for each component in the final composition.

The production of the granular compositions, when the content of imidoalkanepercarboxylic acids in the final granule is less than 30% by weight, may also be performed by direct mixing of all the ingredients by performing step II) simultaneously with step III). In this case, step II) of deposition of the peracids onto the support is performed simultaneously with step III) of mixing and granulation. This process variant may be performed with a batch process or with a continuous process.

When the amount of peracid in the final granule is greater than 30%, the deposition of the peracids onto the support is performed as described in step II).

The drying of the granules in steps II), III) and IV) is performed at temperatures of between 10°C and 65°C and at pressures of between 10 mm Hg and 800 mm Hg.

As mentioned above, the granules may optionally be coated with the binders described above. In this case, after the drying stage, which may be performed on a commercial scale in fluid-bed ovens, the granules may be treated by screening and/or grinding operations to isolate the fractions having the desired particle size, as is known in the field of detergency, and the said fractions are treated by the coating process, which may be performed in batch apparatus or via a continuous process.

It has been found, surprisingly and unexpectedly, by the Applicant that, to obtain the granular compositions based on imidoalkanepercarboxylic acids having the following combination of properties:
■ efficacy under mild application conditions, for example in water at a temperature of from 10°C to 30°C;
■ short wash times, of even less than 30 minutes;
■ dissolution time t₉₀ of the imidoalkanepercarboxylic acids in the washing bath at a temperature of 20°C of less than 10 minutes and preferably less than five minutes;
it is necessary for the said peracids to be deposited onto the support by impregnation or by spraying, an aqueous slurry of the said peracids being fed in.

As mentioned, by mixing the components in dry form and then granulating the mixture obtained according to the techniques of the prior art, for example as described in EP 852 259, it is not possible to obtain granular compositions having the combination of properties indicated above.

The granular compositions obtained by the process of the present invention are used in unmodified form or as a mixture with other ingredients, which may be in granular form, and which are typical of detergency products in granular form or in the form of tablets. The said compositions or the mixtures thereof, as mentioned above, are used for the bleaching and disinfecting applications intrinsic to the field of detergency, for both industrial uses and domestic uses.

The compositions obtained by the process of the present invention are particularly suitable for bleaching, in particular for removing marks from any type of white or coloured fabric, the characteristics of the fabric that has been subjected to the treatment remaining unchanged.

The compositions obtained by the process of the present invention constitute a suitable intermediate for preparing disinfectants, which are particularly valued in the detergency market, precisely for the increasing demand to use mild conditions in washing, which is more and more often performed at low temperature and for short times, which, per se, favour the proliferation of the bacterial load and are harmful to the hygiene. The said disinfectant solutions also find a useful application in the field of the cleaning and sanitization of hard surfaces.

The following examples are given as non-limiting illustrations of the present invention.

### Examples

### Determination of the PAP titre

The analysis is performed by iodometric titration, by titration with sodium thiosulfate of the iodine that is released from the reaction of potassium iodide with the peracid in the composition, according to the following method. An accurately weighed amount of some 500 mg of the formulation is diluted in 100 ml of water, and 10 ml of glacial acetic acid and 30 ml of aqueous 10% w/w potassium iodide solution are then added. The iodine produced from the reaction is titrated with an aqueous sodium thiosulfate solution of known titre, using a Mettler® DL 40 potentiometric titrator equipped with a platinum electrode and a reference electrode.

### Determination of the rate of dissolution of the PAP of a composition in an aqueous solution of a standard detergent base

The rate of dissolution is determined by the following method. A sample of 1000 mg of granules (granulometric fraction from 0.25 mm to 1.40 mm) is dispersed in 3 litres of solution prepared with water with a hardness of 10°F and 5.1 g of standard detergent base, free of bleaching additives (IEC detergent type B, with phosphates - IEC publication 60456), kept stirred and thermostatically regulated at the temperature indicated in the examples (20°C or 40°C). The times at which the samples are taken, measured from the moment of mixing of the two compositions, are plotted on a graph on the x-axis. The times used, expressed in minutes, were as follows: 1, 3, 5, 10, 15, 30, 60, 120. The areas of the PAP peak, determined by HPLC analysis, are plotted on the y-axis of the graph. The times at which the amount of dissolved PAP corresponds, respectively, to 80% (t₈₀), and to 90% (t₉₀) of the total peracid, determined by taking the concentration of PAP obtained asymptotically on the said graph at infinite time as 100%, are determined from the graph obtained.

### Bleaching test

The test is performed by adding 1000 mg of granules (granulometric fraction from 0.25 mm to 1.40 mm) to one litre of 2% sodium carbonate solution coloured with 0.035% of eriochrome T black, leaving the mixture to stand (without stirring) at 20°C for five minutes. The test is positive if the dispersion discolours.

### Stability test

The test is performed by keeping the granules protected from light for seven days at 20°C or at 40°C and at a relative humidity of 75%, the percentage variation of the PAP titre relative to the initial value, which is expressed as a variation in active oxygen (Available Oxygen), being determined at the end of the period.

### Example 1 Comparative

The preparation of PAP-containing granules according to Example 6 of EP 852 259 is repeated.

21.00 kg of PAP powder (titre: 96%, containing 4% PAC, the PAP precursor, produced by the reaction between caprolactam and phthalic anhydride in the presence of water) and 6.60 kg of boric acid are introduced into a 150 litre Loedige® model FKM-150 granulator. The mixture is homogenized for one minute.

3.80 kg of an aqueous solution obtained by mixing 3.60 kg of Acumer® 1510 (polyacrylic acid (PAA) at 25% by weight, molecular weight MW 60 000) and 0.20 kg of Dequest® 2010 (hydroxyethylidenediphosphonic acid, HEDP) are then introduced into the mass, with stirring and with the aid of a chopper, over three minutes.

A wet granulated mass is thus obtained, which is dried in an Aeromatic® fluid bed with a flow of air heated to 60°C. After this drying, screening is performed, to give 24.1 kg of granules of between 0.25 mm and 1.40 mm in size.

The granules obtained comprise the following ingredients, in the weight percentages indicated: 71% PAP, 23% boric acid. The difference to 100% consists of the other minor ingredients that were added during the processing, in the amounts indicated above.

The following tests were performed:
■ stability test for seven days at 20°C: no variation in the PAP titre;
■ bleaching test: NEGATIVE (the solution does not discolour).

The results of the dissolution test, performed at temperatures of 20°C and 40°C, are given in Table 1.

### Example 2 Comparative

Comparative Example 1 is repeated, but introducing into the Loedige® granulator 21.00 kg of PAP powder (titre: 96%, containing 4% of PAC), 5.10 kg of boric acid and 1.50 kg of Randacel® 150 cellulose fibre, a granule disintegrating aid, which is active in aqueous medium. The mixture is homogenized for one minute. 3.80 kg of an aqueous solution obtained by mixing 3.60 kg of Acumer® 1510 and 0.20 kg of Dequest® 2010 are then introduced into the mass, with stirring and with the aid of a chopper, over three minutes.

A wet granulated mass is thus obtained, which is dried in an Aeromatic® fluid bed with a flow of air heated to 60°C. After this drying, screening is performed, to give 23.90 kg of granules of between 0.25 mm and 1.40 mm in size.

The granules obtained comprise the following ingredients, in the weight percentages indicated: 71% PAP, 18% boric acid, 5% cellulose. The difference to 100% consists of the other minor ingredients that were added during the processing, in the amounts indicated above.

The following tests were performed:
■ stability test for seven days at 20°C: no variation in the PAP titre;
■ bleaching test: POSITIVE (the solution discolours).

The results of the dissolution test, performed at temperatures of 20°C and 40°C, are given in Table 1.

### Example 3

In an Aeromatic® fluid bed with a flow of air heated to 60°C are suspended 10.30 kg of Randacel® 150 cellulose fibre, on which are deposited 12.20 kg of PAP, by gradually feeding in 203.30 kg of an aqueous 5% slurry of PAP, the said aqueous slurry being obtained as follows.

11.47 kg of technical-grade PAP of α form, prepared according to patent application WO 2004/007452, 0.30 kg of xanthan gum, 2.00 kg of Hostapur® SAS anionic surfactant and 0.10 kg of DB100 antifoam are dispersed in 188.70 kg of demineralized water at 50°C and ground for five minutes in a Silverson mill, followed by treatment with a mechanical paddle stirrer for 30 minutes at 50°C and then for 30 minutes at room temperature. The composition obtained is an aqueous slurry with an active PAP titre of 5.0%.

The dried product recovered from the Aeromatic® fluid bed is transferred into a 150 litre Loedige® model FKM-150 granulator, into which are then added 5.10 kg of boric acid. The mixture is homogenized for one minute.

3.80 kg of an aqueous solution obtained by mixing 3.60 kg of Acumer® 1510 and 0.20 kg of Dequest® 2010 are then introduced into the mass, with stirring and with the aid of a chopper, over three minutes.

A wet granulated mass is thus obtained, which is dried in an Aeromatic® fluid bed with a flow of air heated to 60°C. After this drying, screening is performed, to give 25.50 kg of granules of between 0.25 mm and 1.40 mm in diameter.

The granules obtained comprise the following ingredients, in the weight percentages indicated: 40% PAP, 18% boric acid, 37% cellulose. The difference to 100% consists of the other minor ingredients that were added during the processing, in the amounts indicated above.

The following tests were performed:
■ stability test for seven days at 20°C: no variation in the PAP titre;
■ stability test for seven days at 40°C and at 75% relative humidity: the PAP titre decreases by 1.7% relative to the initial titre;
■ bleaching test: POSITIVE (the solution discolours).

The results of the dissolution test, performed at temperatures of 20°C and 40°C, are given in Table 1.

### Example 4

In an Aeromatic® fluid bed with a flow of air heated to 60°C are suspended 5.90 kg of Diacel® 150 cellulose fibre, onto which are deposited 16.70 kg of PAP, by gradually feeding in 41.70 kg of an aqueous 40% slurry of PAP, the said aqueous slurry being obtained as follows.

The following components:
■ HEDP Sequion® 10H60 (Bozzetto), 1.30 kg;
■ sodium hydroxide (solution at 50% by weight), 0.30 kg;
■ Genapol® X020 polyethoxylated (2-5 EO) nonionic surfactant (Clariant), 0.40 kg;
are added in order into a reactor containing 60.10 kg of water, the liquid phase being kept stirred by means of a variable-speed motor set at 120 revolutions per minute and equipped with an anchor-stirring shaft.

The solution obtained is heated and maintained at a constant temperature of 45°C.

With stirring, technical-grade PAP in α-crystal form, prepared according to patent application WO 2004/007452, is fed in in an amount of 20.0 kg, via successive additions. During the addition, the mass is kept stirred at a temperature of 45°C and is simultaneously sent for grinding in a colloidal mill. Five minutes after the end of the addition of PAP, the grinding is stopped and stirring is continued for a further 60 minutes.

The temperature of the thermostatic bath is lowered to 20°C and the mass is left for the time required for it to cool down.

At this point, thickening of the solid phase in the lower part of the reactor is observed, 37 kg of clear solution are removed from the upper part of the reactor, to obtain from the lower part 41.7 kg of an aqueous 40% slurry of PAP.

After the addition of this slurry into the Aeromatic® fluid bed is complete, the dried product that is recovered from the fluid bed is transferred into a 150 litre Loedige® model FKM-150 granulator, into which are added 5.10 kg of boric acid. The mixture is homogenized for one minute.

3.80 kg of an aqueous solution obtained by mixing 3.60 kg of Acumer® 1510 and 0.20 kg of Dequest® 2010 are then introduced into the mass, with stirring and with the aid of a chopper, over three minutes.

A wet granulated mass is thus obtained, which is dried in an Aeromatic® fluid bed with a flow of air heated to 60°C. After this drying, screening is performed, to give 26.10 kg of granules of between 0.25 mm and 1.40 mm in size.

The granules obtained comprise the following ingredients, in the weight percentages indicated: 55% PAP, 18% boric acid, 21% cellulose. The difference to 100% consists of the other minor ingredients that were added during the processing, in the amounts indicated above.

The following tests were performed:
■ stability test for seven days at 20°C: no variation in the PAP titre;
■ stability test for seven days at 40°C and at 75% relative humidity: the PAP titre decreases by 1.4% relative to the initial titre;
■ bleaching test: POSITIVE (the solution discolours).

The results of the dissolution test, performed at temperatures of 20°C and 40°C, are given in Table 1.

### Example 5

In an Aeromatic® fluid bed with a flow of air heated to 60°C are suspended 5.90 kg of Diacel® 150 cellulose fibre, onto which are deposited 19.80 kg of PAP, by feeding in 36.00 kg of an aqueous 55% slurry of PAP, the said aqueous slurry being obtained as follows.

The following components:
■ HEDP Sequion® 10H60, 1.50 kg;
■ sodium hydroxide (solution at 50% by weight), 0.40 kg;
■ Genapol® X020 polyethoxylated (2-5 EO) nonionic surfactant, 0.50 kg; are added in order into a reactor containing 71.20 kg of water, the liquid phase being kept stirred by means of a variable-speed motor set at 120 revolutions per minute and equipped with an anchor-stirring shaft.

The solution obtained is heated and maintained at a constant temperature of 45°C.

With stirring, technical-grade PAP in α-crystal form, prepared according to patent application WO 2004/007452, is fed in in an amount of 23.80 kg, via successive additions. During the addition, the mass is kept stirred at a temperature of 45°C and is simultaneously sent for grinding in a colloidal mill. Five minutes after the end of the addition of PAP, the grinding is stopped and stirring is continued for a further 60 minutes.

The temperature of the thermostatic bath is lowered to 20°C, the mass is left for the time required for it to cool down, and it is transferred into a decanting centrifuge, from which are removed 57.40 kg of clear aqueous phase. The thickened slurry contains 55% PAP. 36.00 kg of the said slurry are fed into the fluid bed, as indicated above.

The product suspended in the fluid bed is partially dried and then screened, to give 24.50 kg of granules of between 0.25 and 1.40 mm in size.

The granules thus obtained are removed from the fluid bed, and comprise the following ingredients, in the percentages indicated: 65% PAP, 21% cellulose, 6.5% water. The difference to 100% consists of the other minor ingredients that were added during the processing, in the amounts indicated above.

The following tests were performed:
■ stability test for seven days at 20°C: no variation in the PAP titre;
■ stability test for seven days at 40°C and at 75% relative humidity: the PAP titre decreases by 0.8% relative to the initial titre;
■ bleaching test: POSITIVE (the solution discolours).

The results of the dissolution test, performed at temperatures of 20°C and 40°C, are given in Table 1.

### Comment on Table 1

The table shows that, in the granulates containing PAP deposited on the support (Ex. 3-5), the dissolution times t₈₀ and t₉₀ are appreciably shorter than those for the granules obtained according to the prior art, in which the components, including the peracid, which are all in dry form, are mixed together in bulk in the granulation stage.

The table shows that the dissolution times for the granules according to the present invention are substantially independent of the PAP concentration in the granulate.

**TABLE 1**

| Dissolution times t₈₀ and t₉₀ of PAP of the granular formulations of the examples, each time determined at temperatures of 20°C and 40°C | | | | | | |
|---|---|---|---|---|---|---|
| Ex. | Weight % of PAP in the composition | Granular preparation | T₈₀ (min) | | T₉₀ (min) | |
| | | | 20°C | 40°C | 20°C | 40°C |
| 1 Comparative | 71 | Wet granulation of the components in powder form | 22 | 10 | 30 | 14 |
| 2 Comparative | 71 | As in Comparative Example 1 | 9 | 8 | 14 | 12 |
| 3 | 40 | Deposition of slurry on support followed by granulation | 2.5 | 2.0 | 3.0 | 1.5 |
| 4 | 55 | As in Example 3 | 2.7 | 2.1 | 3.0 | 1.6 |
| 5 | 65 | As in Example 3 | 2.6 | 2.0 | 3.1 | 1.6 |

## Claims

1. Process for obtaining granular compositions of imidoalkanepercarboxylic acids in β form, comprising the said peracids in an amount of from 5 % to 80 % by weight and preferably from 30 % to 70 % by weight; the said peracids being supported on supports with hydrophilic or hydrophobic characteristics; the said granular compositions having a dissolution time t₉₀ of the peracid at 20°C of less than ten minutes and preferably less than five minutes; the sum of the components indicated above and of those optionally present being 100 %, comprising the following steps:
I) preparation of an aqueous dispersion containing an amount of imidoalkanepercarboxylic acids, expressed as a percentage by weight, of from 20 % to 70 %, working at temperatures of between 20°C and 65°C;
II) deposition of the imidoalkanepercarboxylic acids present in the aqueous dispersion prepared in step I) onto the support agent, preferably by impregnation or spraying, using a fluid-bed drier, working at temperatures of between 10°C and 65°C and at a pressure of between 10 mm Hg and 800 mm Hg, to obtain an aggregate;
III) optionally, granulation of the aggregates obtained in step II), optional addition of other additives, and drying of the granules obtained;
IV) optionally, screening of the dried granules, followed by coating of the screened granules and drying of the coated granules;
V) screening of the granules obtained and recovery of the granulometric fraction of between 0.25 mm and 1.40 mm in size.

2. Process according to Claim 1, in which, in step I), the imidoalkanepercarboxylic acids have the formula (I) : 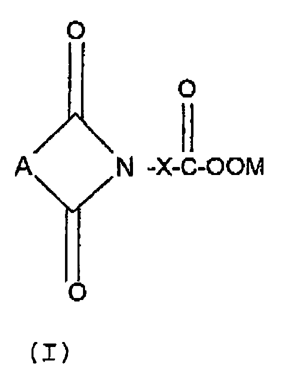 in which A indicates a group chosen from the following : 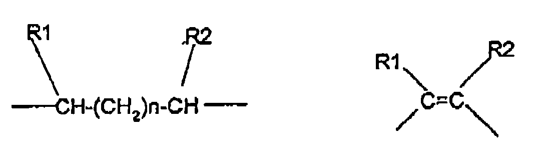 or 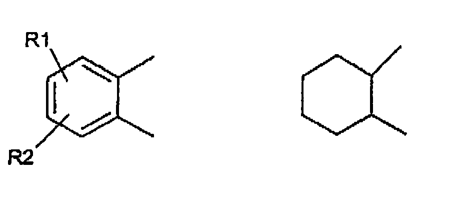 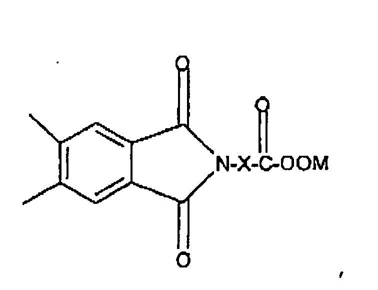 in which:
n is an integer 0, 1 or 2,
R1 has one of the following meanings: hydrogen, chlorine, bromine, C₁-C₂₀ alkyl, C₁-C₂₀ alkenyl, aryl or alkylaryl,
R2 is hydrogen, chlorine, bromine or a group chosen from the following : -SO₃M, -CO₂M, -CO₃M or -OSO₃M,
M means hydrogen, an alkali metal, ammonium or an equivalent of an alkaline-earth metal,
X indicates a C₁-C₁₉ alkylene or an arylene;
the said imidoalkanepercarboxylic acids being in the "β-crystal" form and having a dissolution time t₉₀ in the washing bath at a temperature of 20°C of less than ten minutes, preferably less than five minutes.

3. Process according to Claim 2, in which, in step I), the imidoalkanepercarboxylic acid is ε-phthalimidoperoxyhexanoic acid.

4. Process according to Claims 1 to 3, in which, in step I), imidoalkanepercarboxylic acids in β-crystal form obtained from the corresponding α-crystal form are used.

5. Process according to Claims 1 to 4, in which, in step II), the supports used are substances that are at least partially soluble in the washing baths.

6. Process according to Claim 5, in which, in step II), the supports are from 0.01 mm to 0.35 mm and preferably from amount 0,05 mm to 0,25 mm in size.

7. Process according to Claims 5 and 6, in which, in step II), the supports are natural or synthetic polymers; preferably cellulose, cellulose fibres or methylcellulose, polysaccharides and optionally expanded starch, polyvinylpyrrolidone, natural gums, metal oxides or sulfates, preferably magnesium or aluminium oxide or sulfate, zeolites and silicas.

8. Process according to Claims 5 and 6, in which, in step II), the supports consist of solid components that are added to the composition to control the exothermicity (phlegmatizers).

9. Process according to Claims 5 to 8, in which, in step II), the supports are chosen from cellulose fibres, polysaccharides and starch.

10. Process according to Claims 1 to 9, in which, in step II), the weight ratio between the peracids and the support is between 0.1 and 4.0, the sum of the components of the composition being 100 %.

11. Process according to Claims 1 to 10, in which, in step I), surfactants chosen from anionic and nonionic surfactants, antifoams and suspending agents are used.

12. Process according to Claims 1 to 11, in which, in step II) and in step III), additives for controlling the exothermicity (phlegmatizers), chelating agents and/or sequestering agents, surfactants and binders are also used.

13. Process according to Claim 12, in which the additives for controlling the exothermicity (phlegmatizers) are chosen from boric acid, water and salts of metals from groups IA, IIA and IIIA and of organic or mineral acids that are chemically compatible with the imidoalkanepercarboxylic acids and that can crystallize in hydrated form, chosen from magnesium sulfate pentahydrate, calcium lactate hydrate and calcium sulfate dihydrate; preferably boric acid.

14. Process according to Claim 12, in which the binder is a water-soluble binder of polymer type.

15. Process according to Claims 1 to 14, in which, in step I), viscosifiers are used.

16. Process according to Claims 1 to 15, in which, in step IV), film-forming agents for surface coating are used.

17. Process according to Claims 1 to 16, in which, when the amount of imidoalkanepercarboxylic acids in the granules is not greater than 30 % by weight, step II) is performed simultaneously with step III).

18. Process according to Claims 1 to 17, in which the granules are dried at temperatures of between 10°C and 65°C, at pressures of between 10 mm Hg and 800 mm Hg.

## Patentansprüche

1. Verfahren zur Herstellung von granulatförmigen Zusammensetzungen der Imidoalkanpercarbonsäuren in der β-Form, in denen die genannten Persäuren in einer Menge von 5 bis 80 Gew.-% und bevorzugt 30 bis 70 Gew.-% enthalten sind, wobei die genannten Persäuren auf Trägern mit hydrophilem oder hydrophobem Charakter aufgebracht sind, wobei die genannten granulatförmigen Zusammensetzungen eine Auflösungszeit t₉₀ der Persäure bei 20°C von weniger als zehn Minuten und bevorzugt weniger als fünf Minuten aufweisen und die Summe der oben angegebenen Komponenten und der gegebenenfalls mitenthaltenen 100% beträgt, bei dem man folgende Schritte durchführt:
I) Herstellung einer wäßrigen Dispersion, enthaltend eine Menge der Imidoalkanpercarbonsäuren, ausgedrückt als Gewichtsprozentsatz, von 20% bis 70%, wobei man bei Temperaturen zwischen 20°C und 65°C arbeitet,
II) Abscheidung der in der in Schritt I) hergestellten wäßrigen Dispersion enthaltenen Imidoalkanpercarbonsäuren auf das Trägermittel, bevorzugt durch Imprägnierung oder Sprühen, unter Anwendung eines Wirbelschichttrockners, wobei man bei Temperaturen zwischen 10°C und 65°C und bei einem Druck zwischen 10 mm Hg und 800 mm Hg arbeitet und ein Aggregat erhält,
III) gegebenenfalls Granulierung der in Schritt II) erhaltenen Aggregate, gegebenenfalls Zugabe anderer Zusatzstoffe und Trocknen der erhaltenen Granulate,
IV) gegebenenfalls Sieben der getrockneten Granulate mit anschließender Beschichtung der gesiebten Granulate und Trocknen der beschichteten Granulate,
V) Sieben der erhaltenen Granulate und Gewinnung der granulometrischen Fraktion zwischen 0,25 mm und 1,40 mm Größe.

2. Verfahren nach Anspruch 1, bei dem man in Schritt I) als Imidoalkanpercarbonsäuren solche der Formel (I) einsetzt: 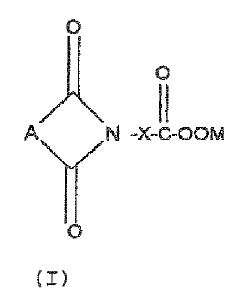 wobei die Variablen jeweils folgende Bedeutung haben:
A steht für eine Gruppe, ausgewählt unter den folgenden: 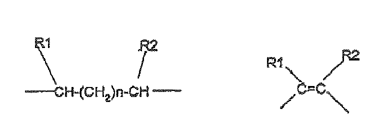 oder 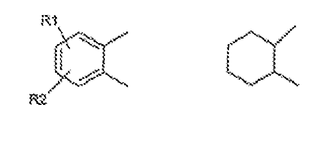 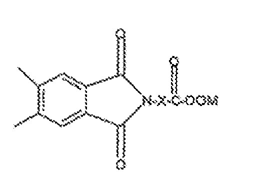 in denen:
n eine ganze Zahl 0, 1 oder 2 bedeutet,
R1 eine der folgenden Bedeutungen hat: Wasserstoff, Chlor, Brom, C₁-C₂₀-Alkyl; C₂-C₂₀-Alkenyl, Aryl oder Alkylaryl,
R2 für Wasserstoff, Chlor, Brom oder eine unter den folgenden: -SO₃M, - CO₂M, -CO₃M oder -OSO₃M ausgewählte Gruppe steht,
M bedeutet Wasserstoff, ein Alkalimetall, Ammonium oder ein Äquivalent eines Erdalkalimetalls,
X bedeutet ein C₁-C₁₉-Alkylen oder ein Arylen,
wobei die genannten Imidoalkanpercarbonsäuren in der β-Modifikation vorliegen und eine Auflösungszeit t₉₀ im Waschbad bei einer Temperatur von 20°C von weniger als zehn Minuten und bevorzugt weniger als fünf Minuten aufweisen.

3. Verfahren nach Anspruch 2, bei dem man in Schritt I) als Imidoalkanpercarbonsäure ε-Phthalimidoperoxyhexansäure einsetzt.

4. Verfahren nach den Ansprüchen 1 bis 3, bei dem man in Schritt I) als Imidoalkanpercarbonsäuren in der β-Modifikation die aus der entsprechenden α-Modifikation erhaltenen einsetzt.

5. Verfahren nach den Ansprüchen 1 bis 4, bei dem man in Schritt II) als Träger Substanzen einsetzt, die sich zumindest teilweise in den Waschbädern auflösen.

6. Verfahren nach Anspruch 5, bei dem man in Schritt II) als Träger solche der Größe 0,01 mm bis 0,35 mm und bevorzugt 0,05 mm bis 0,25 mm einsetzt.

7. Verfahren nach den Ansprüchen 5 und 6, bei dem man in Schritt II) als Träger natürliche oder synthetische Polymere, bevorzugt Cellulose, Cellulosefasern oder Methylcellulose, Polysaccharide und gegebenenfalls aufgeblähte Stärke, Polyvinylpyrrolidon, natürliche Gummen, Metalloxide oder - sulfate, bevorzugt Magnesium- oder Aluminiumoxid oder -sulfat, Zeolithe und Kieselsäuren einsetzt.

8. Verfahren nach den Ansprüchen 5 und 6, bei dem die Träger in Schritt II) aus festen Komponenten bestehen, die der Zusammensetzung als Phlegmatisierungsmittel zur Beherrschung der Exothermie zugesetzt werden.

9. Verfahren nach den Ansprüchen 5 bis 8, bei dem man in Schritt II) die Träger unter Cellulosefasern, Polysacchariden und Stärke auswählt.

10. Verfahren nach den Ansprüchen 1 bis 9, bei dem man in Schritt II) das Gewichtsverhältnis zwischen Persäure und Träger zwischen 0,1 und 4,0 wählt, wobei die Summe der Komponenten der Zusammensetzung 100% beträgt.

11. Verfahren nach den Ansprüchen 1 bis 10, bei dem man in Schritt I) Tenside unter anionischen und nichtionischen Tensiden, Antischaummitteln und Suspendiermitteln auswählt.

12. Verfahren nach den Ansprüchen 1 bis 11, bei dem man in Schritt II) und in Schritt III) auch Zusatzstoffe zur Beherrschung der Exothermie (Phlegmatisierungsmittel), Cheliermittel und/oder Sequestriermittel, Tenside und Bindemittel einsetzt.

13. Verfahren nach Anspruch 12, bei dem man die Zusatzstoffe zur Beherrschung der Exothermie (Phlegmatisierungsmittel) auswählt unter Borsäure, Wasser und mit den Imidoalkanpercarbonsäuren chemisch verträglichen und hydratiert kristallisationsfähigen Salzen von Metallen der Gruppen IA, IIA und IIIA und von organischen oder Mineralsäuren, ausgewählt unter Magnesiumsulfatpentahydrat, Calciumlactathydrat und Calciumsulfatdihydrat, bevorzugt Borsäure.

14. Verfahren nach Anspruch 12, bei dem man als Bindemittel ein wasserlösliches Bindemittel vom Typ Polymer einsetzt.

15. Verfahren nach den Ansprüchen 1 bis 14, bei dem man im Schritt I) Viskosifizierungsmittel einsetzt.

16. Verfahren nach den Ansprüchen 1 bis 15, bei dem man in Schritt IV) Filmbildner für die Oberflächenbeschichtung einsetzt.

17. Verfahren nach den Ansprüchen 1 bis 16, bei dem man bei einem Anteil der Imidoalkanpercarbonsäuren an den Granulaten von höchstens 30 Gew.-% Schritt II) gleichzeitig mit Schritt III) durchführt.

18. Verfahren nach den Ansprüchen 1 bis 17, bei dem man die Granulate bei Temperaturen zwischen 10°C und 65°C und bei Drücken zwischen 10 mm Hg und 800 mm Hg trocknet.

## Revendications

1. Procédé pour l'obtention de compositions granulaires d'acides imidoalcanepercarboxyliques sous forme β, comprenant lesdits peracides en une quantité de 5% à 80% en poids, et de préférence de 30% à 70% en poids; lesdits peracides étant soutenus sur des supports ayant des caractéristiques hydrophiles ou hydrophobes; lesdites compositions granulaires ayant une durée de dissolution t₉₀ du peracide à 20°C, inférieure à 10 minutes et de préférence inférieure à 5 minutes; la somme des composants indiqués ci-dessus et de ceux éventuellement présents faisant 100%, comprenant les étapes suivantes :
I) préparation d'une dispersion aqueuse contenant une quantité d'acides imidoalcanepercarboxyliques, exprimée sous la forme d'un pourcentage en poids, de 20% à 70%, en opérant à des températures comprises entre 20°C et 65°C;
II) dépôt des acides imidoalcanepercarboxyliques présents dans la dispersion aqueuse préparée à l'étape I) sur l'agent support, de préférence par imprégnation ou pulvérisation, en employant un séchoir à lit fluidisé, fonctionnant à des températures comprises entre 10°C et 65°C et à une pression comprise entre 10 mm Hg et 800 mm Hg, afin d'obtenir un agrégat;
III)éventuellement, granulation des agrégats obtenus à l'étape II), éventuelle addition d'autres additifs, et séchage des granulés obtenus;
IV)éventuellement, triage des granulés séchés, suivi par l'enrobage des granulés triés, et séchage des granulés enrobés;
V) triage des granulés obtenus et récupération de la fraction granulométrique comprise entre 0,25 mm et 1,40 mm en taille

2. Procédé selon la revendication 1, dans lequel, à l'étape I), les acides imidoalcanepercarboxyliques possèdent la formule (I) : 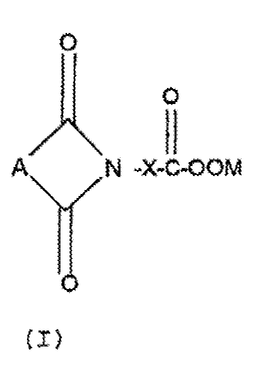 dans laquelle A indique un groupe choisi parmi les suivants : 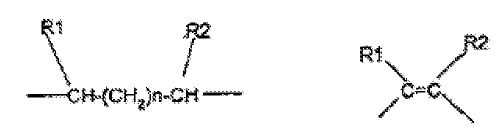 ou 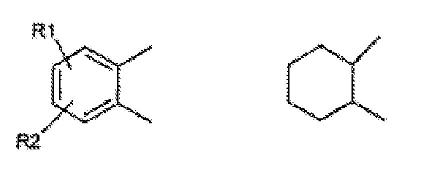 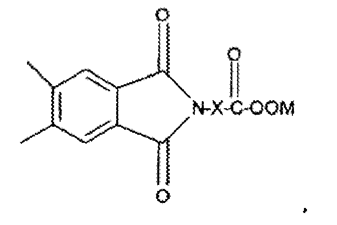 dans lesquels :
n est un nombre entier valant 0, 1 ou 2,
R1 prend l'une des significations suivantes : un atome d'hydrogène, de chlore, de brome, un groupe alkyle en C₁ à C₂₀, alcényle en C₂ à C₂₀, aryle ou alkylaryle,
R2 représente un atome d'hydrogène, de chlore, de brome, ou un groupe choisi parmi les suivants :
-SO₃M, -CO₂M, -CO₃M ou -OSO₃M,
M signifie un atome d'hydrogène, un atome de métal alcalin, l'ion ammonium ou un équivalent d'un métal alcalino-terreux,
X indique un groupe alkylène en C₁ à C₁₉, ou un groupe arylène;
lesdits acides imidoalcanepercarboxyliques se trouvant sous la forme "cristalline β" et possédant une durée de dissolution t₉₀ dans le bain de lavage à une température de 20°C, inférieure à 10 minutes, de préférence inférieure à 5 minutes.

3. Procédé selon la revendication 2, dans lequel, à l'étape I), l'acide imidoalcanepercarboxylique est l'acide ε-phtalimidoperoxyhexanoïque.

4. Procédé selon les revendications 1 à 3, dans lequel sont employés à l'étape I), des acides imidoalcanepercarboxyliques sous la forme cristalline β, obtenus à partir de la forme crystalline α correspondante.

5. Procédé selon les revendications 1 à 4, dans lequel, à l'étape II), les supports employés sont des substances qui sont au moins partiellement solubles dans les bains de lavage.

6. Procédé selon la revendication 5, dans lequel, à l'étape II), les supports ont une taille de 0,01 mm à 0,35 mm, et de préférence de 0,05 mm à 0,25 mm.

7. Procédé selon les revendications 5 et 6, dans lequel, à l'étape II), les supports sont des polymères naturels ou synthétiques; de préférence la cellulose, les fibres de cellulose ou la méthylcellulose, les polysaccharides et éventuellement l'amidon expansé, la polyvinylpyrrolidone, les gommes naturelles, les oxydes ou sulfates métalliques, de préférence l'oxyde ou le sulfate de magnésium ou d'aluminium, les zéolithes et les silices.

8. Procédé selon les revendications 5 et 6, dans lequel, à l'étape II), les supports sont constitués de composants solides que l'on ajoute à la composition afin de maîtriser le caractère exothermique (agents flegmatisants).

9. Procédé selon les revendications 5 à 8, dans lequel, à l'étape II), les supports sont choisis parmi les fibres de cellulose, les polysaccharides et l'amidon.

10. Procédé selon les revendications 1 à 9, dans lequel, à l'étape II), le rapport pondéral entre les peracides et le support est compris entre 0.1 et 4.0, la somme des composants de la composition faisant 100%.

11. Procédé selon les revendications 1 à 10, dans lequel, à l'étape I), sont employés des tensio-actifs choisis parmi les tensio-actifs anioniques et non ioniques, des agents anti-mousse et des agents de suspension.

12. Procédé selon les revendications 1 à 11, dans lequel, à l'étape II), et à l'étape III), sont également employés des additifs pour maîtriser le caractère exothermique (flegmatisants), des agents chélatants et/ou des agents séquestrants, des tensio-actifs et des liants.

13. Procédé selon la revendication 12, dans lequel les additifs pour maîtriser le caractère exothermique (flegmatisants) sont choisis parmi l'acide borique, l'eau et les sels de métaux issus des Groupes IA, IIA et IIIA et d'acides organiques ou minéraux qui sont chimiquement compatibles avec les acides imidoalcanepercarboxyliques et qui peuvent se cristalliser sous une forme hydratée, choisis parmi le sulfate de magnésium pentahydraté, le lactate de calcium hydraté et le sulfate de calcium dihydraté; de préférence l'acide borique.

14. Procédé selon la revendication 12, dans lequel le liant est un liant hydrosoluble du type polymère.

15. Procédé selon les revendications 1 à 14, dans lequel, à l'étape I), sont employés des agents conférant de la viscosité.

16. Procédé selon les revendications 1 à 15, dans lequel, à l'étape IV), sont employés des agents filmogènes pour l'enrobage des surfaces.

17. Procédé selon les revendications 1 à 16, dans lequel, lorsque la quantité des acides imidoalcanepercarboxyliques dans les granulés n'est pas supérieure à 30% en poids, l'étape II) est réalisée simultanément avec l'étape III).

18. Procédé selon les revendications 1 à 17, dans lequel les granulés sont séchés à des températures comprises entre 10°C et 65°C, à des pressions comprises entre 10 mm Hg et 800 mm Hg.
